# EUROPEAN PATENT APPLICATION

(11) **EP 1 683 527 A1**
(43) Date of publication of application: **26.07.2006**
(21) Application number: 04793062.3
(22) Date of filing: 20.10.2004
(51) Int. Cl.: A61K 45/00, A61K 31/7088, A61K 38/17, A61K 48/00, A61P 25/00, A61P 25/28, A61P 43/00

(54) **METHOD OF INHIBITING SECRETASE ACTIVITY**

(30) Priority: 20.10.2003 JP 2003359704
(71) Applicant: Locomogene, Inc., Tokyo 105-0001 (JP)
(72) Inventor: NAKAJIMA, Toshihiro Kohoku Garden Hills A-503, Kanagawa 224-0001 (JP); AMANO, Tetsuya Otaki Haitsu 201 1-21-16, Teraodai, Kanagawa 214-0005 (JP); YAMASAKI, Satoshi Ishikawazaka Manshon 305, Kanagawa 225-0003 (JP); YAGISHITA, Naoko Kosumo Konandai 507, Kanagawa 234-0053 (JP); IKEDA, Rie Haitsu Maria 202 2-31-13, Sugao, Kanagawa 216-0015 (JP); ZHANG, Lei Sankopo Yamana 102 6-9-20, Komagome, Tokyo 170-0003 (JP)
(74) Representative: Woods, Geoffrey Corlett
(86) International application number: PCT/JP2004/015950
(87) International publication number: WO 2005/037316

(57) **Abstract**

The present invention provides a method for inhibiting secretase activity. A method for promoting the sensitivity of a secretase inhibitor, a method for binding synoviolin to Herp (homocysteine-inducible endoplasmic reticulum stress-inducible ubiquitin-like domain member 1), or the like is employed to inhibit secretase activity.

## Description

### TECHNICAL FIELD

The present invention relates to a method for inhibiting secretase activity and a pharmaceutical composition thereof. Specifically, it relates to a method for inhibiting secretase activity by inhibiting synoviolin expression, thereby promoting the sensitivity of a secretase inhibitor, to a method for inhibiting secretase activity by binding synoviolin to Herp, and to a pharmaceutical composition comprising a substance that inhibits secretase activity.

### BACKGROUND ART

Synoviolin is a novel protein that was discovered as a membrane protein in synovial cells derived from rheumatoid arthritis patients (WO 02/05207). Since studies using genetically modified animals have shown that this factor is directly involved in bone and joint development and in the development of joint disease, synoviolin is thought to be a protein whose activity contributes to normal bone formation or limb development.

Because synoviolin is expressed ubiquitously not only in the bones and joints but throughout the body, a search for factors which bind with synoviolin is effective as a means of analyzing the functions of synoviolin in vivo. In particular, if the substrate protein of synoviolin could be discovered, it would provide an important key for identifying the intracellular signaling pathways associated with synoviolin.

Therefore, in order to elucidate which intracellular signaling pathways are associated with synoviolin, the present inventors searched for factors that bind to synoviolin using the Yeast Two Hybrid method with synoviolin as Bait. As a result, a protein called Herp (homocysteine-inducible endoplasmic reticulum stress-inducible ubiquitin-like domain member 1) was identified as a molecule that interacts with synoviolin. Herp is a protein that was discovered in 1996 by Miyata et al as a product of a homocysteine-respondent gene in vascular endothelial cells (Kokame K, Kato H, Miyata T, "Homocysteine-respondent Genes in Vascular Endothelial Cells Identified by Differential Display Analysis," *J. Biol Chem.* 1996, Nov. 22, 271 (47): 29659-29665).

Subsequent research has shown that Herp is a protein which structurally has a ubiquitin-like domain (UBL) at the N-terminal, and that its expression is induced by endoplasmic reticulum stress (Kokame K, Agarwala KL, Kato H, Miyata T, "Herp, a New Ubiquitin-like Membrane Protein Induced by Endoplasmic Reticulum Stress," *J. Biol. Chem.* 2000, Oct. 20, 275(42): 32846-32853). It has also been reported that Herp elevates the activity of γ-secretase, a proteolytic enzyme which interacts with presenilin (PS) (believed to be a causal gene in familial Alzheimer's disease (FAD)) and is involved in intramembrane cleavage of the β-amyloid protein (Aβ) (Sai X, Kawamura Y, Kokame K, Yamaguchi H, Shiraishi H, Suzuki R, Suzuki T, Kawaichi M, Miyata T, Kitamura T, Strooper BD, Yanagisawa K, Komano H, *J*. *Biol. Chem.* 277(15): 12915-12920).

Alzheimer's disease is a disease that has given more concern as the population ages at the present time. Its primary character is to be shown the deposition of senile plaque or in other words fibrous β-amyloid protein (Aβ) in the brain. The β-amyloid protein itself is formed when the amyloid precursor protein (APP) is cleaved by β-secretase and γ-secretase, and this cleavage is elevated in a patients of Alzheimer's disease.

Consequently, substances that inhibit the enzyme activity of secretases are being screened worldwide as potential therapeutic agents for Alzheimer's disease.

However, although several candidate substances have been obtained, no secretase inhibitor has yet been successfully developed.

### DISCLOSURE OF THE INVENTION

It is an object of the present invention to provide a therapeutic agent useful for treatment of cerebro-neurological diseases, in particular, Alzheimer's disease.

As a result of exhaustive research aimed at solving the aforementioned problems, the inventors focused on a substance that inhibits secretase activity, and arrived at the present invention upon finding that Aβ accumulation could be suppressed by the said substance and Alzheimer's disease treated using this substance.

That is, the present invention is as follows.
(1) A pharmaceutical composition comprising a substance that inhibits secretase activity.
(2) The pharmaceutical composition according to (1), wherein the secretase is β-secretase or γ-secretase.
(3) The pharmaceutical composition according to (1) or (2), wherein the substance that inhibits secretase activity is a substance that promotes the sensitivity of a secretase inhibitor.
(4) The pharmaceutical composition according to (3), wherein the substance that promotes the sensitivity of a secretase inhibitor is a substance that inhibits expression of synoviolin.
(5) The pharmaceutical composition according to (4), wherein the substance that inhibits expression of synoviolin is siRNA or shRNA for a gene coding for synoviolin.
(6) The pharmaceutical composition according to (5), wherein the gene coding for synoviolin comprises the nucleotide sequence represented by SEQ ID NO: 1.
(7) The pharmaceutical composition according to (5), wherein the siRNA targets a part of the nucleotide sequence represented by SEQ ID NO: 1.
(8) The pharmaceutical composition according to (7), wherein the part of the nucleotide sequence is at least one selected from the nucleotide sequences represented by SEQ ID NOS 3-16.
(9) The pharmaceutical composition according to (1) or (2), wherein the substance that inhibits secretase activity is synoviolin. The synoviolin may be for example synoviolin having the amino acid sequence represented by SEQ ID NO: 2.
(10) The pharmaceutical composition according to any one of (1) through (9) for treating a cerebro-neurological disease.
(11) The pharmaceutical composition according to (10), wherein the cerebro-neurological disease is Alzheimer's disease.
(12) A method for inhibiting secretase activity wherein the sensitivity of a secretase inhibitor is promoted.
(13) The method according to (12), wherein the sensitivity of a secretase inhibitor is promoted by inhibiting the expression of synoviolin.
(14) A method for inhibiting secretase activity wherein synoviolin is bound to Herp.
(15) The method according to (14), wherein the binding region of Herp with synoviolin is the region represented by the amino acid residues Nos. 161-200 in the amino acid sequence of Herp.
(16) The method according to any one of (12) through (16), wherein the secretase is β-secretase or γ-secretase.

### BRIEF DESCRIPTION OF THE DRAWINGS

Figure 1 is a schematic view showing the full-length structure of synoviolin and the structures of the Syno-dTM and Syno-Ring used as Bait.
Figure 2 is a schematic view showing the full-length structure of Herp and the structures of fragmented Herp.
Figure 3 shows the results of tests of binding between Flag-Syno dTM and GST fusion proteins with various Herp constructs.
Figure 4 shows the results of an immunoprecipitation test of HA/Syno with Flag/Herp.
Figure 5 shows the effects of a γ-secretase inhibitor in wild-type and synoviolin-deficient mouse embryonic fibroblasts.
Figure 6 shows the relationship between synoviolin and Herp in a pull down assay.
Figure 7 shows the binding regions of synoviolin and Herp in vivo.
Figure 8A shows Herp expression in MEF cells.
Figure 8B shows ubiquitination of Herp.
Figure 8C shows the constructs used to analyze Herp.
Figure 9 shows γ-secretase activity in MEF cells.

### BEST MODE FOR CARRYING OUT THE INVENTION

The present invention is explained in detail below.

Focusing on the possibility that synoviolin might be involved in the pathogenetic mechanism of Alzheimer's disease, the inventors showed that the sensitivity of a secretase inhibitor was elevated in cells in which synoviolin expression was knocked out. This means that inhibiting synoviolin expression promotes the sensitivity of the secretase inhibitor, indicating that the sensitivity of the secretase inhibitor is promoted via synoviolin deficiency. This is evidence that a substance that promotes the sensitivity of a secretase inhibitor could be used to treat Alzheimer's disease.

The inventors also discovered that when synoviolin is made to bind to the Herp protein, the Herp protein is ubiquitinated and broken down, and secretase activity declines as a result.

### 1. Outline

As described above, in Alzheimer's disease, APP is cleaved by a secretase (such as γ-secretase), and Aβ accumulates, forming senile plaque. Focusing on the involvement of secretase in the pathogenetic mechanism of Alzheimer's disease, the inventors therefore considered a construction of a process which Aβ accumulation and senile plaque formation could be suppressed by inhibiting secretase activity.

Hence, the present invention is characterized by that Aβ accumulation is suppressed by inhibiting secretase activity. By suppressing Aβ accumulation, it is possible to inhibit formation of senile plaques and treat Alzheimer's disease.

Synoviolin is a protein that is expressed throughout the body and has been shown to control essential functions in the body. Consequently, it is necessary that the functions of synoviolin in vivo are elucidated. In general, a useful method for elucidating the functions of a protein is to identify factors that bind to said protein, and, specifically, if the substrate protein of synoviolin could be discovered, it would provide an important key for identifying the intracellular signaling pathways associated with synoviolin.

Therefore, in order to elucidate the intracellular signaling pathways associated with synoviolin, the inventors in this case performed of a binding test in yeast and a GST Pull-down assay, as described above, to show that synoviolin interacts with Herp. When the inventors caused synoviolin and Herp to be co-expressed in HEK293 cells and observed them by co-precipitation in order to confirm the intracellular interactions, synoviolin and Herp were seen to interact as they did in yeast and in vitro assay. This supports the idea that synoviolin interacts with Herp. A protein structural prediction system indicated the presence of a RING finger motif in synoviolin. This motif is known to exist in E3 ubiquitin-protein ligase, which is involved in breaking down proteins. Moreover, the RING finger motif is thought to be a binding site for E2 ubiquitin-conjugating enzyme.

Consequently, synoviolin is thought to be involved in the pathogenetic mechanism of Alzheimer's disease and in secretase activity in particular.

In the present invention, promotion of the sensitivity of a secretase inhibitor and binding of synoviolin and the Herp protein (hereinafter sometimes called simply "Herp") were considered as possible mechanisms for inhibiting the activity of secretase.

In the first case, promoting the sensitivity of a secretase inhibitor suppresses secretase activity, thereby inhibiting accumulation of Aβ, while in the second case binding of synoviolin to Herp causes ubiquitination of Herp, breaking down Herp and thereby inhibiting accumulation of Aβ.

These respective modes are discussed below.

### 2. Promoting the sensitivity of a secretase inhibitor

Focusing on the sensitivity of a secretase inhibitor, the inventors thought of constructing a process by which accumulation of Aβ and formation of senile plaque could be inhibited by increasing the sensitivity of the inhibitor. Considering the possibility that synoviolin might be involved in the pathogenetic mechanism of Alzheimer's disease, the sensitivity of a secretase inhibitor was shown to be higher in cells in which synoviolin expression was knocked out. This means that inhibiting synoviolin expression promotes the sensitivity of the secretase inhibitor, indicating that the sensitivity of the secretase inhibitor is promoted via synoviolin deficiency. This is evidence that a substance that promotes the sensitivity of a secretase inhibitor could be used to treat Alzheimer's disease.

"Promoting the sensitivity of a secretase inhibitor" here means that the pharmacological effects of the secretase inhibitor are enhanced so that it functions more effectively.

### (1) Inhibiting synoviolin expression and activity

A method for inhibiting expression of synoviolin is adopted for increasing the sensitivity of a secretase inhibitor.

There are no particular limits on the means of inhibiting synoviolin expression, but for example RNA interference (RNAi) can be used. siRNA (small interfering RNA) for the synoviolin gene can be designed and synthesized, and introduced into cells to cause RNAi.

RNAi is a phenomenon in which dsRNA (double-stranded RNA) binds specifically and selectively to a target gene, cleaving the target gene and thereby efficiently inhibiting its expression. For example, when dsRNA is introduced into cells expression of a gene having a sequence homologous to that RNA is suppressed (knocked out).

siRNA can be designed as follows.
(a) There are no limits on the gene as long as it codes for synoviolin, and all regions are potential candidates. For example, in the case of humans any region of GenBank Accession number AB024690 (SEQ ID NO: 1) is a potential candidate.
(b) A sequence starting with AA is selected from the chosen region, and this sequence is 19 to 25 or preferably 19 to 21 nucleotides in length. A sequence can be selected which has a GC content of 40 to 60% for example. Specifically, of the nucleotide sequence represented by SEQ ID NO: 1, DNA comprising the following nucleotide sequences can be used as the target sequence for the siRNA. In particular, (i) (SEQ ID NO: 3), (ii) (SEQ ID NO: 4), (vi) (SEQ ID NO: 8), (vii) (SEQ ID NO: 9) and (viii) (SEQ ID NO: 10) can be targeted by preference.
   (i) AA TGTCTGCATCATCTGCCGA GA (SEQ ID NO: 3)
   (ii) AA GCTGTGACAGATGCCATCA TG (SEQ ID NO: 4)
   (iii) AA AGCTGTGACAGATGCCATC AT (SEQ ID NO: 5)
   (iv) AA GAAAGCTGTGACAGATGCC AT (SEQ ID NO: 6)
   (v) AA GGTTCTGCTGTACATGGCC TT (SEQ ID NO: 7)
   (vi) AA CAAGGCTGTGTACATGCTC TA (SEQ ID NO: 8)
   (vii) AA ATGTTTCCACTGGCTGGCT GA (SEQ ID NO: 9)
   (viii) AA GGTGTTCTTTGGGCAACTG AG (SEQ ID NO: 10)
   (ix) AA CATCCACACACTGCTGGAC GC (SEQ ID NO: 11)
   (x) AA CACCCTGTATCCAGATGCC AC (SEQ ID NO: 12)
   (xi) AA GGTGCACACCTTCCCACTC TT (SEQ ID NO: 13)
   (xii) AA TGTTTCCACTGGCTGGCTG AG (SEQ ID NO: 14)
   (xiii) AA GAGACTGCCCTGCAACCAC AT (SEQ ID NO: 15)
   (xiv) AA CGTTCCTGGTACGCCGTCA CA (SEQ ID NO: 16)

A method such as linking siRNA synthesized in vitro to plasmid DNA and introducing it into cells or a method for annealing two strands of RNA can be employed for introducing the siRNA into the cells.

shRNA can also be used in the present invention to produce an RNAi effect. shRNA, called short hairpin RNA, is an RNA molecule having a stem loop structure so that some region of the single strand can form a complementary strand with another region.

shRNA can be designed so that part of it forms a stem loop structure. For example, given sequence A of one region and sequence B which is complementary to sequence A, the shRNA can be designed with a total length of 45 to 60 nucleotides so that these sequences are included in one strand of RNA in the order of sequence A, spacer, sequence B. Sequence A is the sequence of a partial region of the target synoviolin gene (SEQ ID NO: 1), and all regions are potential candidates, with no particular limits on the target region. Sequence A is 19 to 25 or preferably 19 to 21 nucleotides in length.

### (2) Secretase inhibition activity

Since there are ethical objections to sensitivity measurement tests using the brains of Alzheimer's patients, another evaluation system needs to be used for evaluating the sensitivity of the secretase inhibitor. The inventors therefore used an evaluation system in which cells were treated with a secretase inhibitor and cell proliferation was used as the marker of secretase inhibition. There are no particular limits on the type of secretase here, and examples include β-secretase and γ-secretase. Consequently, the secretase inhibitor can be either a β-secretase inhibitor or a γ-secretase inhibitor. There are no particular limits on the secretase inhibitor, and examples include L-685,458 (Peptide Institute), (3,5-Difluorophenylacetyl)-Ala-Phg-OBu^{t} [DAPT] (Peptide Institute), Lys-Thr-Glu-Glu-Ile-Ser-Glu-Val-Asn-Sta-Val-Ala-Glu-Phe (Peptide Institute), Z-Leu-Leu-Nle-CHO (Wako) and the like.

Secretase activity is judged to have been inhibited when cell proliferation activity is suppressed. That is, the more cell proliferation activity is suppressed, the greater the secretase inhibition effect and the sensitivity. Using cells which are obtained from mouse embryonic fibroblasts (MEF) having the wild-type synoviolin gene and synoviolin-deficient mice in which the synoviolin gene had been knocked out, the change of sensitivity of the secretase activity with or without the expression of synoviolin is compared.

When the effects of the inhibitor were evaluated using the aforementioned synoviolin-deficient cells, cell proliferation was lower in the cells lacking synoviolin than in the wild-type cells having synoviolin. That is, by lacking synoviolin, the sensitivity of a secretase inhibitor is promoted.

### (3) Pharmaceutical composition

The shRNA and siRNA prepared in the present invention are substances which inhibit expression of synoviolin, and can be used as a pharmaceutical composition comprising a substance which promotes the sensitivity of a secretase inhibitor (particular a gene therapy agent for Alzheimer's disease and other cerebro-neurological disorders).

When the pharmaceutical composition of the present invention is used as a gene therapy agent for a cerebro-neurological disorder, it is targeted at the brain (cerebrum, interbrain, mid-brain, cerebellum), medulla oblongata, spinal cord and other parts of the central nervous system.

There may be one or more than one of the aforementioned cerebro-neurological disorders.

When the pharmaceutical composition of the present invention is used as a gene therapy agent, it can be administered directly by injection, or a vector with incorporated nucleic acid can be administered. Examples of the aforementioned vector include adenovirus vectors, adeno-associated virus vectors, herpes virus vectors, vaccinia virus vectors, retrovirus vectors, lentivirus vectors and the like, and efficient administration can be accomplished using these virus vectors.

The pharmaceutical composition of the present invention can also be introduced into a liposome or other phospholipid microsome, and that microsome can then be administered. A microsome bearing siRNA or shRNA is introduced into a specific cell by lipofection. The resulting cells can then be administered by general administration either intravenously, arterially or the like. Local administration to the brain or the like is also possible.

The dosage of the pharmaceutical composition of the present invention differs depending on age, sex, symptoms, administration route, number of administrations and agent formulation, but in the case of an adenovirus for example the dosage is about 10⁶ to 10¹³ cells once a day, administered at intervals of 1 to 8 weeks.

A commercial gene introduction kit (such as AdenoExpress (Clontech)) can be used to introduce siRNA or shRNA into a target tissue or organ.

### 3. Binding of synoviolin and Herp

As discussed above, Herp is an endoplasmic reticulum transmembrane protein which structurally has a UBL at the N-terminal, expression of which is induced by endoplasmic reticulum stress, and endogenous cellular Herp is known to be polyubiquitinated. Focusing on Herp, the inventors thought of constructing a process in which Aβ accumulation and senile plaque formation could be inhibited by breaking down this Herp, thereby suppressing γ-secretase activity and inhibiting intramembrane cleavage of Aβ.

Also, as discussed above, when the yeast two hybrid method using synoviolin as Bait was used to search for factors binding to synoviolin as discussed above, synoviolin was shown to interact with Herp, and this interaction was also observed in cells. The inventors therefore investigated a method for inhibiting secretase activity by binding synoviolin to Herp.

### (1) Determining the minimal binding site of Herp with synoviolin

The minimal binding site of Herp with synoviolin can be determined by a binding test using synoviolin and various cleaved fragments of Herp. A synoviolin binding test using Herp showed that the minimal binding site of Herp with synoviolin is the region of the 40 amino acid residues Nos. 161-200 in the amino acid sequence (SEQ ID NO: 18) encoded for by the human Herp gene (SEQ ID NO: 17) (following sequence).
GYTPYGWLQLSWFQQIYARQYYMQYLAATAASGAFVPPPS (SEQ ID NO: 19)

The synoviolin used in the present invention may have the amino acid sequence (SEQ ID NO: 2) encoded for by the aforementioned SEQ ID NO: 1, or may have the aforementioned amino acid sequence having 1 or more amino acids deleted, replaced or added and having the ability to break down Herp by ubiquitination. In the present invention, the aforementioned deletions, substitutions or other mutations may be introduced by a known site-directed mutagenesis method, using for example a GeneTailor™ Site-Directed Mutagenesis System (Invitrogen) or TaKaRa Site-Directed Mutagenesis System (Mutan-K, Mutan-Super Express Km or the like) (Takara).

The Herp protein region necessary for binding with synoviolin in cultured cells can be identified as follows.

For human Herp, by preparing a Herp(-bind) gene lacking the region of the aforementioned 40 amino acid residues, inserting this gene into an appropriate vector, and introducing the vector into HEK293T or other cells to induce strong expression together with synoviolin, it is possible to determine whether the deleted site is a site essential for binding between synoviolin and Herp. The N-terminal of this Herp(-bind) can be labeled with a HA tag for purposes of an immunoprecipitation test, and the synoviolin can be labeled in the same way with a FLAG tag.

The results can be analyzed by extracting whole cell extract (WCE) after strong expression, confirming expression of the protein, and then immunoprecipitating with anti-HA antibodies or anti-FLAG antibodies. When the resulting immunoprecipitate is tested by Western blotting, Herp binds with synoviolin but Herp(-bind) does not, showing that the region of amino acids Nos. 161 through 200 of the Herp protein (SEQ ID NO: 19) is essential for binding with synoviolin.

### (2) Confirming decomposition of Herp by ubiquitination

When Herp binds with synoviolin, it is ubiquitinated and broken down. It can be confirmed whether or not Herp is the substrate of synoviolin by polyubiquitination of Herp. That is, a Herp mutant with a FLAG tag attached and some of the amino acid residues mutated is prepared in HEK293T cells, and HA-Ubiquitin and synoviolin expression is induced. Next, whole cell extract (WCE) is prepared, expression of each protein is confirmed by Western blotting, and immunoprecipitation is performed with anti-HA antibodies to confirm whether or not Herp is polyubiquitinated (poly-Ubiquitin; Ub) in cells.

In the Herp wild type, a polyubiquitination band is observed when ubiquitin alone is strongly expressed or when ubiquitin and synoviolin are strongly expressed simultaneously. Also, the polyubiquitination bands of the Herp and Herp mutant are weaker when ubiquitin and synoviolin are strongly expressed simultaneously than when ubiquitin alone is strongly expressed, indicating greater decomposition of polyubiquitinated Herp. In the Herp(-bind), no polyubiquitination band is detected either when ubiquitin alone is strongly expressed or when ubiquitin and synoviolin are strongly expressed at the same time. In the case of Herp(-UBL), which is Herp with the ubiquitin region (UBL) deleted, the Herp(-UBL) polyubiquitination band is stronger when ubiquitin and synoviolin are strongly expressed at the same time than when ubiquitin alone is strongly expressed. This shows that the UBL region of Herp is not essential for binding with synoviolin or for polyubiquitination by synoviolin, but is essential for decomposition.

### (3) Comparison of γ-secretase activity in MEF cells

The involvement of synoviolin in Alzheimer's disease can be studied using synoviolin knockout cells (for example MEF (syno(-/-)) by comparing γ-secretase activity in wild-type cells (for example, MEF (syno(+/+)). That is, MEF cells are treated with lysis buffer to prepare a cell lysate, a reaction buffer and fluorescent-labeled substrate are then added to accomplish an enzyme reaction, and fluorescence can then be measured at a specific excitation wavelength and measurement wavelength to confirm whether or not γ-secretase activity is elevated in the MEF cells.

In the present invention, when synoviolin is made to bind to Herp, Herp is broken down and γ-secretase activity can be inhibited. Herp is also known to interact with presenilin (PS), which is believed to be a causative gene of FAD, forming a complex and thereby to increase γ-secretase activity. This is evidence that Alzheimer's disease could be treated using a binding product of synoviolin with Herp and a binding product of synoviolin with a Herp-PS complex.

### (4) Pharmaceutical composition

In the present invention, synoviolin is a substance involved in inhibiting secretase activity and can be used in a pharmaceutical composition for treating Alzheimer's disease and other cerebro-neurological disorders because when synoviolin binds to Herp or a Herp-PS complex, the Herp is broken down by ubiquitination, thus inhibiting the accumulation of Aβ protein, which is a cause of Alzheimer's disease.

When the pharmaceutical composition of the present invention is used as a therapeutic agent for cerebro-neurological disorders, it is applied to the brain (cerebrum, interbrain, mid-brain, cerebellum), medulla oblongata, spinal cord and other parts of the central nervous system. The pharmaceutical composition of the present invention can be applied directly to an affected part, or can be introduced into the target cells or organs by all the known way, for example, intravenous, intramuscular, intraperitoneal, subcutaneous or other injection, by inhalation via the nasal cavity, oral cavity or lungs, by oral administration or by intravenous administration using a catheter or the like for example.

It can also be made easier to handle by freezing or the like, and then be used directly or mixed with known excipients, extenders, binders, lubricants and other pharmacologically acceptable carrier and known additives (including buffers, tonicity agents, chelating agents, colorants, preservatives, perfumes, flavorings, sweeteners and the like).

The pharmaceutical composition of the present invention may be administered orally or parenterally in an oral form such as tablets, capsules, powder, granules, pills, liquid, syrup or the like or in a parenteral form such as an injection, external application, suppository, eye drops or the like. Preferably, it is injected locally by intramuscular or intraperitoneal injection or the like or else injected into the veins or the like.

The dosage is determined appropriately according to the kind of effective ingredient, the administration route, the object of administration, the patient's age, weight, sex, symptoms or other conditions, but the daily maintenance dose is about 0.1 to about 100 mg or preferably 0.1 mg to 10 mg or more preferably 0.1 mg to 1.0 mg per kg for an adult. Synoviolin can be administered once a day or can be administered in multiple administrations.

When the pharmaceutical composition (synoviolin) of the present invention is made to be expressed in vivo (gene therapy), the synoviolin gene can be administered directly by injection, or a vector into which the synoviolin gene has been incorporated can be administered as discussed above in reference to gene therapy agents. Examples of the aforementioned vector include adenovirus vectors, adeno-associated virus vectors, herpes virus vectors, vaccinia virus vectors, retrovirus vectors, lentivirus vectors and the like, and efficient administration can be accomplished using these vectors.

The synoviolin gene used in the present invention need not have the nucleotide sequence represented by SEQ ID NO: 1 above, and may be a gene which hybridizes under stringent conditions with a sequence complementary to that nucleotide sequence and which codes for a protein which has binding activity to Herp. "Stringent conditions" mean conditions of salt concentration 100 to 500 mM or preferably 150 to 300 mM and temperature 50 to 70°C or preferably 55 to 65°C in the washing process during hybridization.

The synoviolin gene can also be introduced into a liposome or other phospholipid microsome for purposes of administration. A microsome bearing the aforementioned gene is introduced into a specific cell by lipofection. The resulting cell can then be administered by general administration either intravenously, arterially or the like. Local administration to the brain or the like is also possible.

The dosage of the synoviolin gene differs depending on age, sex, symptoms, administration route, number of dosages a day, agent formulation or the like, but in the case of an adenovirus, for example, the dosage is about 10⁶ to 10¹³ cells once a day, administered at intervals of 1 to 8 weeks.

A commercial gene introduction kit (such as AdenoExpress (Clontech)) can be used for introducing the synoviolin gene into the target tissue or organ.

The present invention is explained in detail below using examples. However, the present invention is not limited by these examples.

### [Example 1]

This example is a working example of Herp screening using the Yeast Two Hybrid system.

A MATCHMAKER system (CLONTECH) was used for the Yeast Two Hybrid system, which employed the yeast transformation method (*Pro. Natl. Aca. Sci. USA,* 88: 9578-9582, 1991).

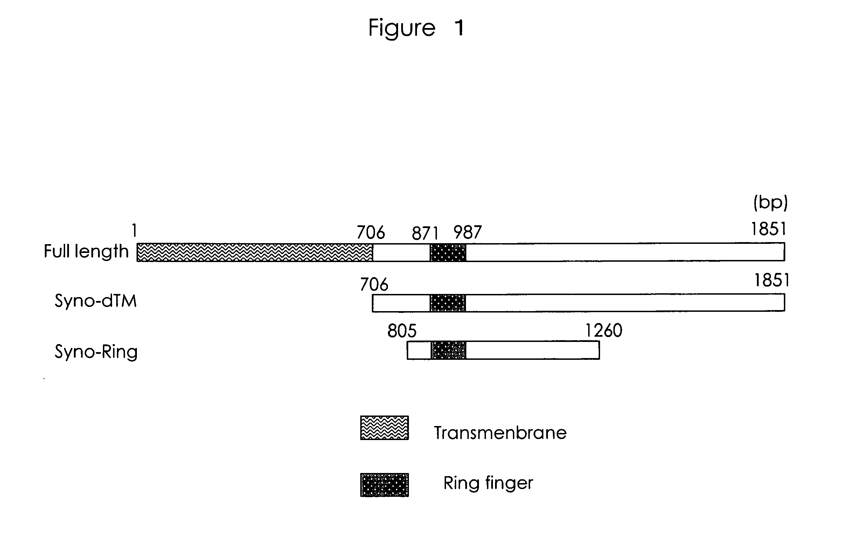

The end of synoviolin cDNA from 706 bp (amino acid #236) to 1851 bp (amino acid #617) or from 805 bp (amino acid #269) to 1260 bp (amino acid #420) was inserted into the Eco R I/Xho I site of a pGBT9 vector as EcoR I/Xho I (from 706 bp (amino acid #236) to 1851 bp (amino acid #617) of synoviolin is hereinafter called Syno dTM, while from 805 bp (amino acid #269) to 1260 bp (amino acid #420) is called Syno Ring: see Figure 1). In Figure 1, "bp" indicates a position in the nucleotide sequence.

The library used for Herp screening consisted of human cartilage-derived cDNA inserted into a pACT2 vector (pACT2-Y). Following 15 minutes of heat shock at 42°C, pGBT9-Syno dTM or pGBT9-Syno Ring (2.0 µg) were inserted into yeast strain Y190 together with pACT2-Y (20 µg).

The Y190 strains with the inserted each vector was washed with Tris-EDTA (pH 7.5) buffer (TE), and a solution of Y190 diluted with TE was spread on an SD-Trp-Leu-His plate and cultured for 10 days at 30°C. The resulting colonies were subjected to a β-galactosidase filter assay using 0.5 mg/ml of X-gal as the substrate, and positive clones were detected.

The positive clones were shaking cultured in SD-Leu-His medium at 30°C for 10 days, and plasmid DNA was extracted by the alkali-SDS method (*Methods Enzymol.,* 194:169-182, 1991). The extracted plasmid DNA was transformed into *E*. *coli* strain HB101, spread on an M9 plate (-Leu), and cultured for 2 days at 37°C. The resulting colonies were shaking cultured for 16 hours at 37°C in 20 µg/ml LB medium, and plasmid DNA was extracted by the alkali-SDS method.

A BigDye Terminator Cycle Sequencing System (Applied Biosystems) was used to analyze the inserted human cartilage-derived cDNA fragments in the extracted plasmid DNA. When the sequence analysis results were searched by BLAST, the homocysteine-inducible endoplasmic reticulum protein "homocysteine-inducible endoplasmic reticulum stress-inducible ubiquitin-like domain member 1" (Accession No. BC032673, hereinafter called "Herp") was obtained.

### [Example 2]

In this example, synoviolin was bound to Herp in vitro.

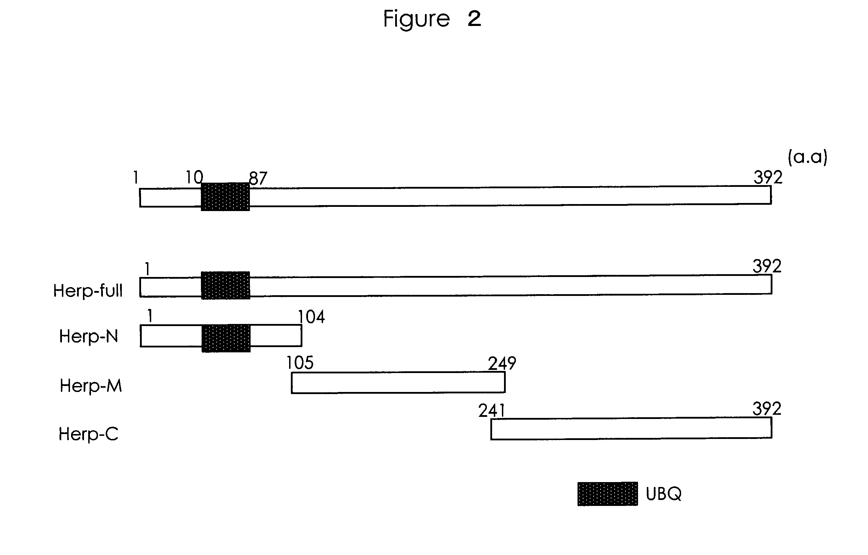

pcDNA3-Flag-Syno dTM was prepared with synoviolin from 706 bp to 1854 bp and a TNT-coupled Translation System (Promega) inserted, and subjected to in vitro translation to prepare (i) a Flag-Syno dTM fusion protein, (ii) a Herp GST fusion protein, and (iii) a GST fusion protein with fragmented Herp (Figure 2). In Figure 2, "a.a." shows the position in the amino acid sequence, while UBQ indicates a ubiquitin domain.

These proteins and (iv) GST protein as a control were added to a binding buffer comprising 20 mM Hepes (pH 7.9), 100 mM NaCl, 1 mM EDTA, 0.05% Tween, 5% Glycerol, 1 mM DTT, 0.2 mM NaVO₄, 5 mM NaF and 1 mM PMSF, and reacted for 16 hours at 4°C. The reaction product was subjected to SDS-PAGE, and radioactivity was detected with an image analyzer (BAS2000, Fujix).

As a result, binding was observed between the Herp-M and Herp-C GST fusion proteins and the [³⁵S]pcDNA3-FlagSyno dTM.

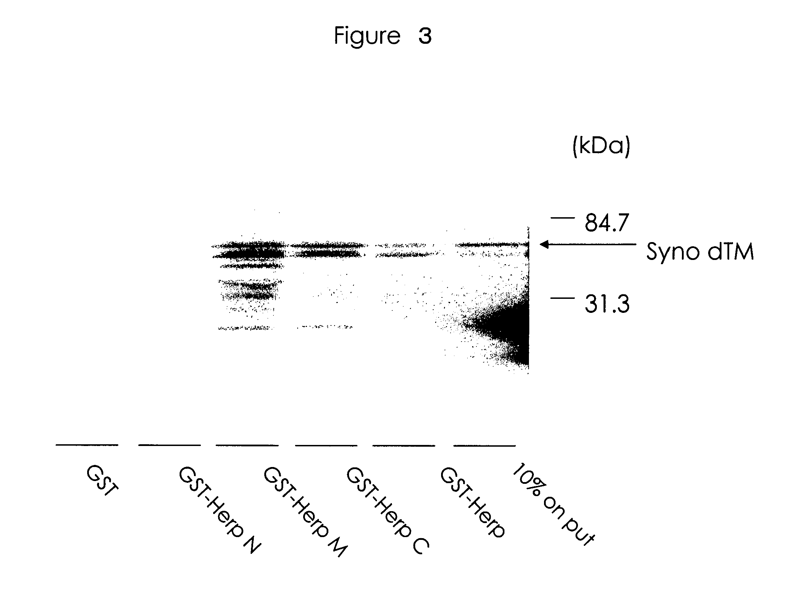

No binding occurred between the control GST and the pcDNA3-FlagSyno dTM (Figure 3). These results suggest that synoviolin and Herp bind through protein interaction.

### [Example 3]

In this example, synoviolin and Herp were bound in vivo.

2 x 10⁶ HEK293 cells were inoculated on a 10 cm dish and cultured for 24 hours, and 3 µg each of pcDNA3-HA/Syno with synoviolin from 1 bp to 1854 bp inserted and pcDNA3-Flag/Herp with Herp from 1 bp to 1176 bp inserted were introduced into the cells. 24 hours after gene introduction the cells were collected and lysed with lysis buffer, and immunoprecipitation was performed overnight at 4°C with anti-HA and anti-Flag antibodies. The bound proteins and free proteins were separated by centrifugation, and detected by Western blotting. Anti-HA and anti-Flag antibodies were used for detection.

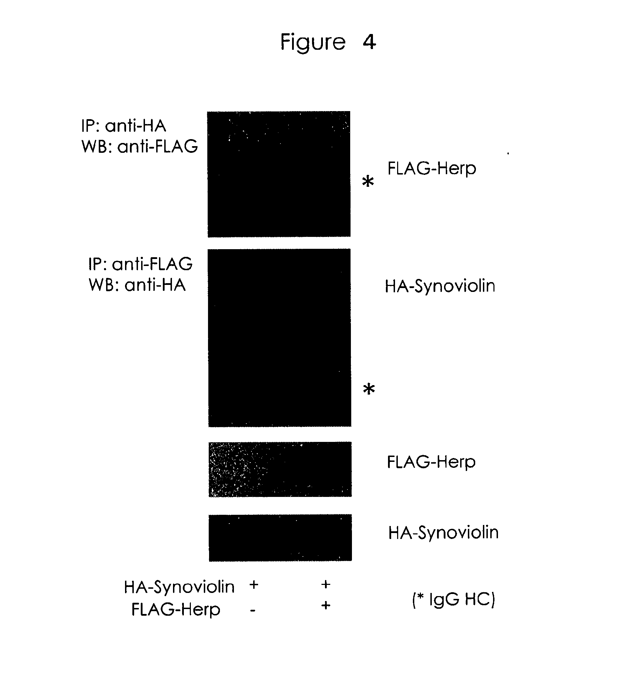

As a result, a Flag-Herp band was confirmed by detection with anti-Flag antibodies following immunoprecipitation with anti-HA antibodies in the cells co-expressing synoviolin and Herp. Moreover, an HA-synoviolin band was confirmed by detection with anti-HA antibodies following immunoprecipitation using anti-Flag antibodies. These results suggest that synoviolin and Herp bind by protein interaction as they do in vitro (Figure 4). In Figure 4, IP indicates the kind of antibody used in immunoprecipitation, while WB indicates the kind of antibody used in Western blotting. An asterisk* indicates the IgG HC (IgG heavy chain).

### [Example 4]

In this example, the effects of a γ-secretase inhibitor on cell proliferation activity in synoviolin-deficient cells were confirmed.

Embryonic fibroblasts (MEFs) obtained from synoviolin gene-introduced mice (wild-type mice) and synoviolin gene-deficient mice were inoculated to 1 x 10³ cells/well on 96-well plates, and cultured overnight. After culture the cells were treated with a γ-secretase inhibitor (Z-Leu-Leu-Nle-CHO, Wako) and cultured for 24 hours, after which Alamer Blue was added and absorbance was measured after 2 hours.

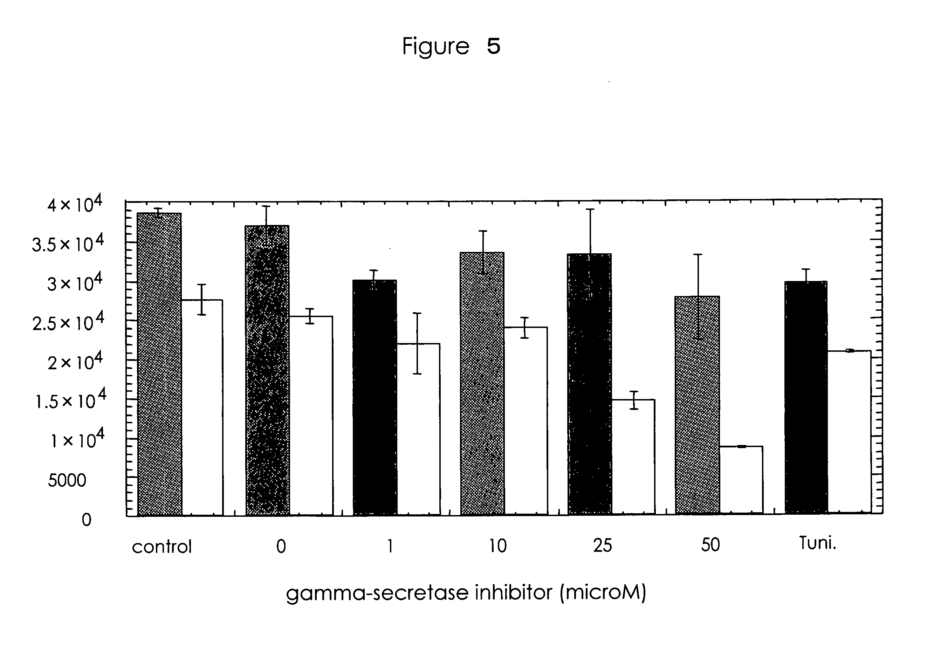

The results are shown in Figure 5. In Figure 5, the black column indicates the cells derived from wild-type mice, while the white column indicates the cells derived from synoviolin-deficient mice. The effect of the γ-secretase inhibitor was greater in the cells derived from synoviolin-deficient mice than in the cells derived from wild-type mice. Tuni. represents tunicamycin.

Figure 5 shows that when γ-secretase activity was inhibited in embryonic fibroblasts derived from synoviolin-deficient mice (Syno -/-), cell proliferation activity was inhibited more than in embryonic fibroblasts derived from synoviolin mice (Syno +/+). This means that that the sensitivity of cells to γ-secretase inhibitors varies depending on the presence or absence of synoviolin, or in other words that the sensitivity of a γ-secretase inhibitor is promoted in the absence of synoviolin. Consequently, a synoviolin inhibitor can be used as a therapeutic agent for Alzheimer's disease to regulate the accumulation of Aβ, which is considered to be the first stage in the pathology of Alzheimer's disease.

### [Example 5]

In this example, the minimal binding site of Herp for synoviolin is determined.

That is, the minimal binding site for synoviolin on Herp, a substrate candidate molecule for synoviolin obtained from a human cartilage cDNA library, was determined by Yeast Two Hybrid Screening with synoviolin as Bait. In other words, fragmented inserts were prepared by PCR using the template Herp incorporated into a pcDNA3 Flag or HA vector. Synoviolin and Herp were fragmented and inserted into pGEX and pcDNA3 vectors for purposes of GST Pull-down assay.

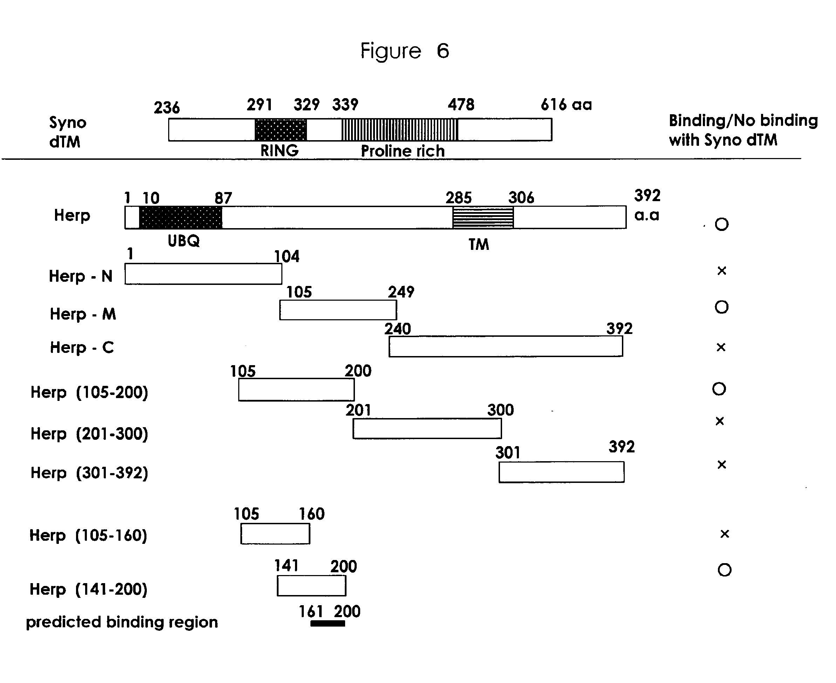

As a result, it was predicted that Herp amino acid sequence Nos. 161-200 would bind with syno dTM (Figure 6).

### [Example 6]

In this example, the Herp protein region necessary for binding with synoviolin in cultured cells was identified.

From the results of Example 5, it appears that the Herp amino acid sequence necessary for binding between synoviolin and Herp consists of the 40 amino acids 161-200 (Figure 6). Therefore, Herp(-bind) was prepared lacking the 40 amino acids 161-200, and used in a synoviolin binding test in cells.

It has been shown that Herp binds with synoviolin, and progress has been made in identifying the region on the Herp protein which binds with synoviolin (Nos. 161-200 in amino acid sequence). In this example, the following test was performed to determine the effect on binding between Herp and synoviolin when this region was deleted.

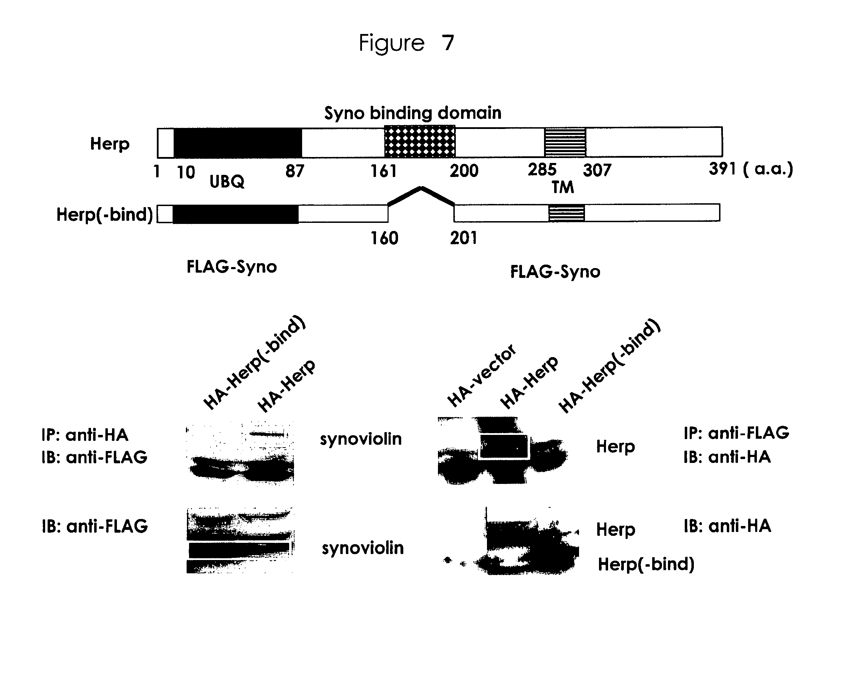

The part of the Herp gene that codes for amino acids Nos. 161-200 was deleted by PCR to prepare a Herp(-bind) gene (Figure 7, top). An HA tag was attached to the N-terminal of this gene, which was inserted into a pcDNA3 vector. Next, Ha-tagged Herp, Herp(-bind) and a blank vector were each strongly expressed together with FLAG-tagged synoviolin in HEK293T cells. After 24 hours whole cell extract (WCE) was extracted, expression of each protein was confirmed, and immunoprecipitation was performed with anti-HA antibodies or anti-FLAG antibodies.

When the immunoprecipitate was washed with wash buffer comprising 150 mM NaCl and subjected to Western blotting, it was found that while Herp binds to synoviolin, Herp(-bind) does not (Figure 7, bottom). This shows that the region of amino acids Nos. 161-200 on the Herp protein is essential for binding with synoviolin.

### [Example 7]

This example tested that Herp is the substrate for synoviolin.

The experimental methods are as follows.

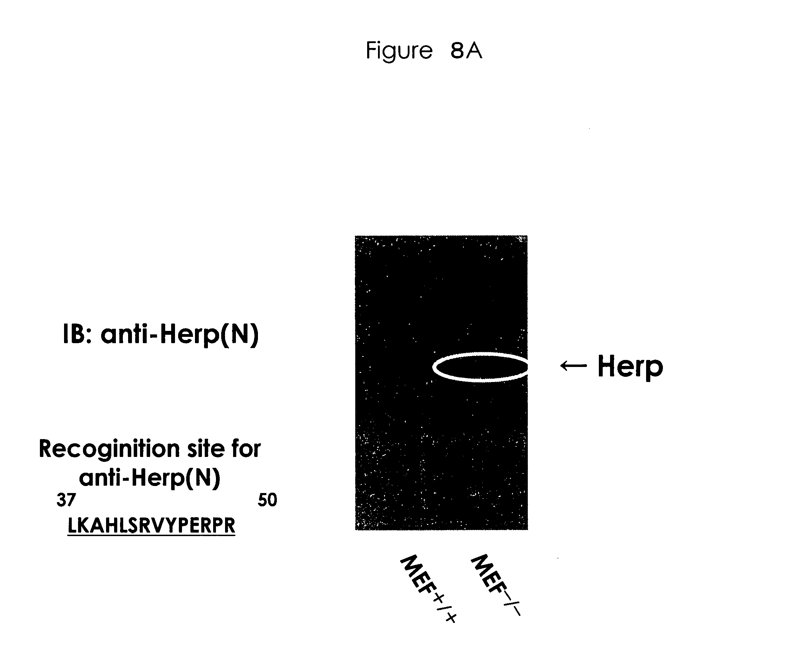

It was shown by yeast two-hybrid and GST pull-down testing that Herp binds with synoviolin in cells. Herp is an endoplasmic reticulum transmembrane protein having an UBL at the N-terminal, and Herp has been reported to be polyubiquitinated when expressed in cells (Kokame, K, Kato H and Miyata T, "Homocysteine-respondent Genes in Vascular Endothelial Cells Identified by Differential Display Analysis," *J. Biol. Chem.* 1996, Nov. 22: 271(47): 29659-29665). It has also been observed that the Herp protein accumulates more in synoviolin (-/-) MEF cells than it does in synoviolin (+/+) MEF cells (Figure 8A). Consequently, there is a strong possibility that Herp is the substrate for synoviolin.

In the aforementioned tests, an amino acid sequence consisting of the region of amino acids Nos. 37-50 in the Herp sequence (LKAHLSRVYPERPR: SEQ ID NO: 20) was used as the recognition site for the anti-Herp (N) antibody.

In an in vitro ubiquitin reaction system, however, a ubiquitination band was observed even in the absence of synoviolin in the case of GST-Herp, which is a GST fusion protein of Herp.

Therefore, this example studied whether Herp is a substrate of synoviolin which undergoes self-ubiquitination.

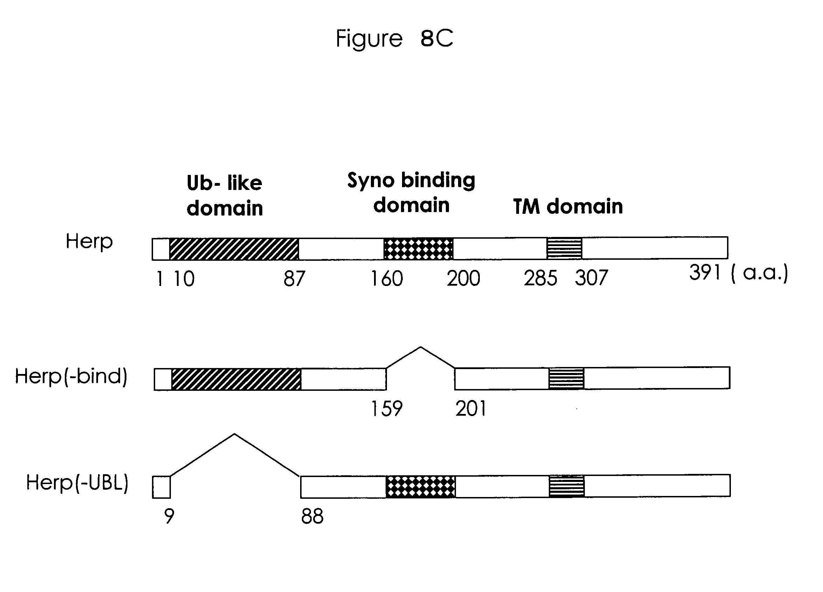

Different FLAG-tagged Herp mutant constructs (Figure 8C) were made to be strongly expressed together with HA-Ubiquitin/pcDNA3 or synoviolin/pcDNA3 in HEK293T cells.

24 hours after strong expression, whole cell extract (WCE) was extracted from the cells, and expression of each protein was confirmed by Western blotting. Immunoprecipitation was also performed with anti-HA antibodies, and polyubiquitination of the Herp protein was tested using anti-FLAG antibodies.

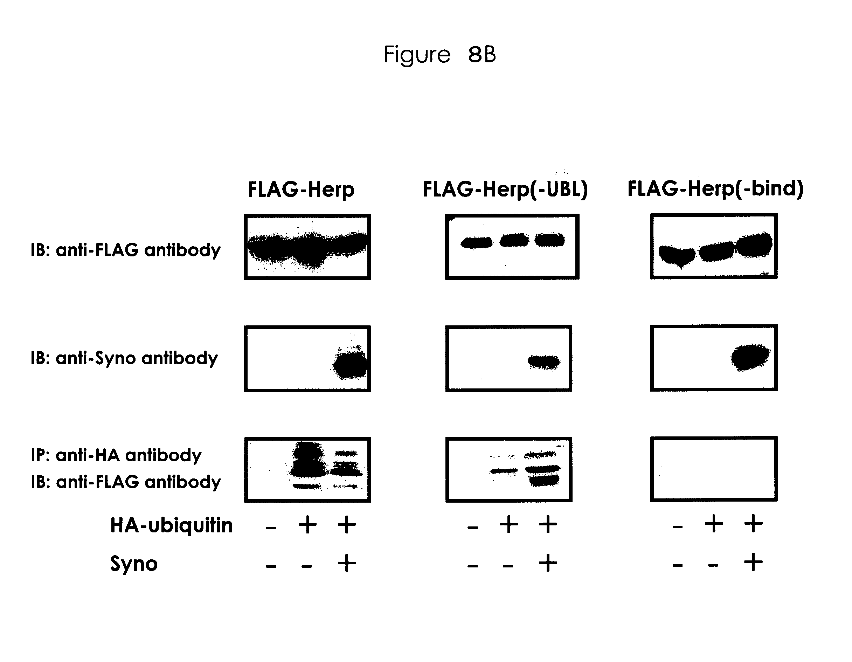

In the Herp wild type, a polyubiquitination band was observed when ubiquitin alone was strongly expressed or when ubiquitin was strongly expressed at the same time as synoviolin. The Herp ubiquitination band was also weaker when ubiquitin and synoviolin were strongly expressed at the same time than when ubiquitin alone was strongly expressed, suggesting greater decomposition of ubiquitinated Herp. By contrast, no polyubiquitination band was detected when ubiquitin alone was strongly expressed or when ubiquitin and synoviolin were strongly expressed at the same time in Herp(-bind). In the case of Herp(-UBL), the ubiquitination band was stronger when ubiquitin and synoviolin were strongly expressed at the same time than when ubiquitin alone was strongly expressed (Figure 8B). This suggests that the UBL region of Herp is essential for decomposition but not for binding with synoviolin or for ubiquitination by synoviolin.

### [Example 8]

In this example, γ-secretase activity in MEFs (syno (+/+) and (-/-)) was compared in order to study the involvement of synoviolin in Alzheimer's disease.

MEFs (syno (+/+) and (-/-)) were treated with lysis buffer to prepare cell lysates to which reaction buffer was added together with a fluorescent-labeled substrate (synthetic APP), and an enzyme reaction was performed for 2 hours at 37°C. Fluorescence was measured at an excitation wavelength of 360 nm and a measurement wavelength of 465 nm.

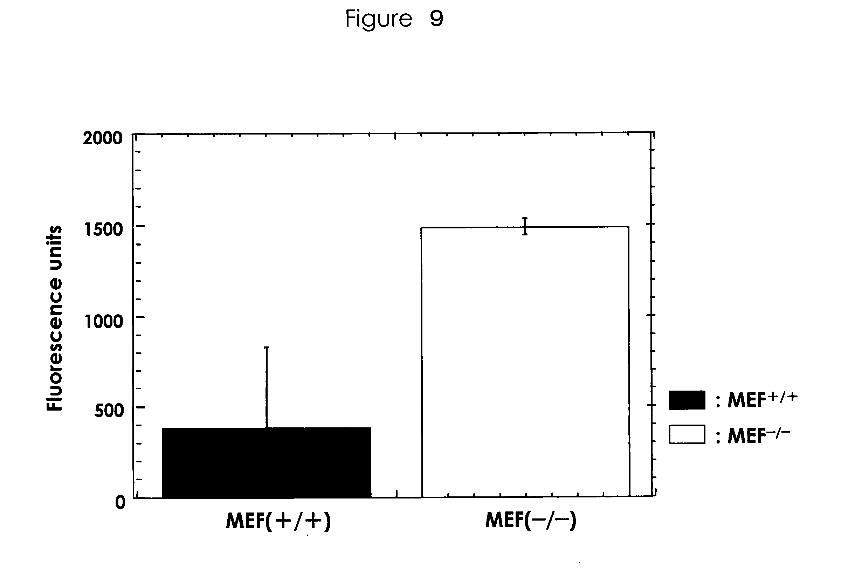

As a result, γ-secretase activity was greater in syno (+/+) than in syno (-/-) MEF (Figure 9).

### INDUSTRIAL APPLICABILITY

The present invention provides a method for inhibiting secretase activity. The present invention also provides a pharmaceutical composition comprising a substance that inhibits secretase activity. The pharmaceutical composition of the present invention is useful as a therapeutic agent for Alzheimer's disease and other cerebro-neurological disorders.

## Claims

1. A pharmaceutical composition comprising a substance that inhibits secretase activity.

2. The pharmaceutical composition according to Claim 1, wherein the secretase is β-secretase or γ-secretase.

3. The pharmaceutical composition according to Claim 1 or 2, wherein the substance that inhibits secretase activity is a substance that promotes the sensitivity of a secretase inhibitor.

4. The pharmaceutical composition according to Claim 3, wherein the substance that promotes the sensitivity of a secretase inhibitor is a substance that inhibits expression of synoviolin.

5. The pharmaceutical composition according to Claim 4, wherein the substance that inhibits expression of synoviolin is siRNA or shRNA for a gene coding for synoviolin.

6. The pharmaceutical composition according to Claim 5, wherein the gene coding for synoviolin comprises the nucleotide sequence represented by SEQ ID NO: 1.

7. The pharmaceutical composition according to Claim 5, wherein the siRNA targets part of the nucleotide sequence represented by SEQ ID NO: 1.

8. The pharmaceutical composition according to Claim 7, wherein the part of the nucleotide sequence is at least one selected from the nucleotide sequences represented by SEQ ID NOS 3-16.

9. The pharmaceutical composition according to Claim 1 or 2 wherein the substance that inhibits secretase activity is synoviolin.

10. The pharmaceutical composition according to any one of Claims 1 through 9 for treating a cerebro-neurological disease.

11. The pharmaceutical composition according to Claim 10, wherein the cerebro-neurological disease is Alzheimer's disease.

12. A method for inhibiting secretase activity wherein the sensitivity of a secretase inhibitor is promoted.

13. The method according to Claim 12, wherein the sensitivity of a secretase inhibitor is promoted by inhibiting expression of synoviolin.

14. A method for inhibiting secretase activity wherein synoviolin is bound to Herp.

15. The method according to Claim 14, wherein the binding region of Herp with synoviolin is the region represented by amino acid residues Nos. 161-200 in the amino acid sequence of Herp.

16. The method according to any one of Claims 12 through 15, wherein the secretase is β-secretase or γ-secretase.
